# EUROPEAN PATENT APPLICATION

(11) **EP 1 669 340 A1**
(43) Date of publication of application: **14.06.2006**
(21) Application number: 04773684.8
(22) Date of filing: 01.10.2004
(51) Int. Cl.: C07C 45/36, C07C 47/54, C07C 47/565, C07C 67/39, C07C 69/78, C07C 69/84

(54) **METHOD FOR OXIDIZING AROMATIC COMPOUND HAVING ALKYL SUBSTITUENT, METHOD FOR PRODUCING AROMATIC ALDEHYDE COMPOUND AND METHOD FOR PRODUCING AROMATIC CARBOXYLIC ACID ESTER**

(30) Priority: 02.10.2003 JP 2003344363
(71) Applicant: NIPPON SHOKUBAI CO., LTD., Osaka-shi, Osaka 541-0043 (JP)
(72) Inventor: HAYASHI, Toshio, c/o Nippon Shokudai Co., Ltd., Suita-shi, Osaka 564-8512 (JP)
(74) Representative: Müller-Boré & Partner Patentanwälte
(86) International application number: PCT/JP2004/014882
(87) International publication number: WO 2005/033055

(57) **Abstract**

The present invention relates a method for oxidation of an aromatic compound having an alkyl substituent, including reacting the aromatic compound having an alkyl substituent with an oxygen molecule to oxidize the alkyl substituent into an aldehyde group in the presence of a catalyst containing Ag and/or Au, and, if necessary, any one or more kinds of group VIII elements, supported on a carrier. The oxidation method of the present invention allows the production of an aromatic aldehyde compound or an aromatic carboxylic acid ester via this aromatic aldehyde compound in high yield and in high selectivity by the use of a catalyst having moderate oxidizing ability, even when an aromatic compound having an alkyl substituent, which is easily converted into an aromatic carboxylic acid by oxidation, is used as a starting material.

## Description

### TECHNICAL FIELD

The present invention relates to a method for oxidizing an alkyl substituent into an aldehyde group using an aromatic compound having an alkyl substituent, and oxygen as starting materials, and further relates to a method for producing an aromatic aldehyde compound and an aromatic carboxylic acid ester using this oxidation method.

### BACKGROUND ART

Conventional methods for producing aromatic aldehyde compounds, particularly methods for oxidizing aromatic compounds having an alkyl substituent with oxygen to give aromatic aldehydes, have been difficult to produce the aromatic aldehydes in high yield and in high selectivity. This may probably be due to the reason that the aldehydes as products can easily undergo sequential oxidation, by which they are oxidized into aromatic carboxylic acids. Therefore, aromatic aldehyde compounds have heretofore been produced as by-products in the production of aromatic carboxylic acids, or produced by other oxidation methods or by other reactions from other starting materials.

For example, conventional methods for producing hydroxy benzaldehydes generally include ordinary methods based on the Reimer-Tiemann reaction in which phenol as a starting material is reacted with chloroform in a sodium hydroxide / methanol solution (e.g., see U.S. Patent No. 3,365,500), and ordinary methods for converting phenol into hydroxybenzyl dichloride by chlorine, followed by hydrolysis (see Japanese Patent Publication No. 47-49046). However, these methods, although they have high reaction yield, have serious problems such that by-products are formed in large quantity or the purification step becomes complicated.

Furthermore, as methods for producing hydroxy benzaldehydes by direct oxidation of cresols with oxygen, there have been proposed, for example, a method of using a catalyst containing copper and cobalt (see Chemical Communication 2002, 622), and a method of vapor phase oxidation using a metal oxide catalyst (see Japanese Patent Laid-Open Publication No. 1-100141). However, it is the present situation that these methods cannot give sufficient yield for industrial production. In addition, there has also been proposed a method for oxidation with oxygen using a compound of cobalt and the like, as a catalyst, and using a great amount of a base (NaOH or the like). However, this method has the serious problem that by-product salts are produced in large quantity in the process (see U.S. Patent No. 4, 453, 016 and Angew. Chem. Intern. Edit. , 14, 356 (1975) 2) .

On the other hand, as methods for production of aromatic carboxylic acid esters, there are ordinary methods for esterifying aromatic carboxylic acids by reaction with alcohols. For example, in the industrial method for production of hydroxybenzoic acid esters, the hydroxybenzoic acid esters are produced by way of multi-stage reactions in which phenol is reacted with carbon dioxide in the presence of an alkali to produce an alkali salt of hydroxybenzoic acid (the Kolbe-Schmitt reaction) and this product is reacted with a strong acid to give a free hydroxybenzoic acid, which is then esterified by reaction with alcohols. In addition to the serious problem that this method for production needs multi-stage reaction steps, it has the serious problem to be solved that by-product salts are produced in large quantity.

Thus, in order to solve these serious problems in the prior art, the present invention has an objective to obtain an aromatic aldehyde compound or an aromatic carboxylic acid ester in high yield and in high selectivity, by the use of an aromatic compound having an alkyl substituent as a starting material, and by the finding of catalysts, solvents, and optimal reaction conditions for carrying out oxidation with high efficiency in the oxidation of the alkyl substituent with oxygen.

### DISCLOSURE OF THE INVENTION

The present invention relates to a method for oxidation of an aromatic compound(s) having an alkyl substituent(s), comprising reacting the aromatic compound(s) having an alkyl substituent(s) with an oxygen molecule to oxidize the alkyl substituent (s) into an aldehyde group(s) in the presence of a catalyst containing Ag and/or Au supported on a carrier.

### BEST MODE FOR CARRYING OUT THE INVENTION

The present invention has a technical feature that an aromatic compound having an alkyl substituent is reacted with an oxygen molecule to oxidize the alkyl substituent into an aldehyde group in the presence of a catalyst containing Ag and/or Au supported on a carrier. That is, as described above, alkyl substituents can easily be oxidized into carboxyl groups by oxidation with oxygen, but a method for oxidation using a catalyst of the present invention makes it possible to stop the oxidation at the stage of aldehyde groups. Therefore, in other words, a method for oxidation of an aromatic compound having an alkyl substituent according to the present invention can be said to be a method for producing an aromatic aldehyde compound. In addition, as described later, only the presence of a primary alcohol as a reaction solvent and a reaction partner in the above oxidation makes it possible to further produce an aromatic carboxylic acid ester from the aromatic aldehyde compound. Even in the method of the present invention, the technical feature is in that the alkyl substituent of an aromatic compound is oxidized into an aldehyde group with the above catalyst.

As the oxidation by the use of a catalyst containing gold (Au) , there have been known some reactions in which alcohols are oxidized to produce carbonyl compounds such as aldehydes and ketones. For example, there has been reported a method of synthesizing aromatic aldehydes by oxidation with oxygen, using aromatic alcohols as starting materials (see Appl. Catal. A: General 211, 251 (2001)). The method of the present invention is, however, essentially different from these oxidations and has excellent general-purpose properties, in that not aromatic alcohols but aromatic compounds having an alkyl substituent are used as starting materials and the alkyl substituent moiety is oxidized to produce aromatic aldehyde compounds.

The present invention will be described below in detail. In the following description, the term "yield" means the yield of a desired product and corresponds to a value calculated by multiplying a conversion (i.e., a ratio in percentage of the starting material converted into different compounds (reaction products), also referred to as activity) in a selectivity (i.e., a ratio in percentage of a desired product in the reaction products).

### 1. Oxidation catalyst

The catalyst used in the oxidation of the present invention is a catalyst containing Ag and/or Au supported on a carrier. The form of Ag and/or Au supported on a carrier is not particularly limited, and any kinds of forms, so long as the desired catalyst performance is exhibited, can be used. The Ag and/or Au may preferably be supported on the surface of a carrier in the form of metal particles. The amount of the above metal supported on a carrier is 0.01% by mass to 30% by mass, preferably 0.1% by mass to 10% by mass, relative to the carrier. When the amount of the above metal supported on a carrier is smaller than 0.01% by mass, catalytic performance cannot sufficiently be exhibited. Even when the above metal is supported on a carrier at an amount of greater than 30% by mass, no improvement in catalytic activity is observed, which is not preferred from the viewpoint of costs.

A more preferred catalyst is a catalyst containing Ag and/or Au, and any one or more kinds of group VIII elements, supported on a carrier. The term group VIII element means Fe, Co, Ni, Ru, Rh, Pd, Os, Ir, and Pt, and the use of a catalyst containing any one or more kinds of these elements, and Ag and/or Au, supported on a carrier may further increase yield and conversion. Au is more preferred in catalytic activity when compared between Ag and Au. In the group VIII elements, Ru, Rh, Pd, Ir, and Pt are preferred, and Pd is most preferred.

Also when Ag and/or Au are used in combination with any one or more kinds of group VIII elements, they may preferably be supported in the form of metal particles on the surface of a carrier. As for the metal particles, metal particles containing Ag and/or Au, and metal particles containing any one or more kinds of group VIII elements, may separately be supported on a carrier, and it is more preferred that metal particles containing both Ag and/or Au and any one or more kinds of group VIII elements are supported on a carrier. That is, it is preferred from the viewpoint of catalytic performance that the metal particles are supported on a carrier as metal particles containing an alloy or an intermetallic compound of Ag and/or Au, and any one or more kinds of group VIII elements, and a larger ratio of such metal particles in all the metal particles provides better effect.

The metal particles may preferably be metal fine particles having an average particle diameter of not greater than 10 nm, more preferably not greater than 6 nm. The lower limit of the average particle diameter is not particularly limited, but it may approximately be 1 nm from the viewpoint of physical stability. The average particle diameter of the metal fine particles indicates an arithmetic mean value of particle diameters of the remaining 80% of particles after excluding both the portion of 10% in the sequential order from larger to smaller particles, and the portion of 10% in the sequential order from smaller to larger particles in 300 or more particles arbitrarily selected from the particles on a carrier by observation with a transmission electron microscope (TEM). In addition, the particle size distribution of the metal fine particles may preferably have a local maximum value of 1 to 10 nm. The local maximum value may more preferably have an upper limit of 6 nm, still more preferably 5 nm. A narrower distribution of the particle diameters is more preferred, and the standard deviation of the particle diameters of the above 300 or more particles may preferably be not greater than 2, particularly preferably not greater than 1.5.

When any one or more kinds of group VIII elements are used in combination with Ag and/or Au, the metal elements may also be supported on a carrier at an amount of 0.01% to 30% by mass, preferably 0.1% to 10% by mass, relative to the carrier, as the total amount of the metal elements to be used.

The composition of Ag and/or Au and any one or more kinds of group VIII elements in the catalyst is not particularly limited, but when Ag and/or Au are defined at the ratio of 1, the ratio of a group VIII element (when two or more kinds of group VIII elements are used, the total amount thereof) may preferably be in the range of 0.01 to 100, more preferably in the range of 0.1 to 10, by atomic ratio.

The catalyst of the present invention may contain any other elements than the above metal elements in such a range that catalytic performance is not affected. For example, the other elements include transition elements of group V to VII, typical elements of group IB to VB, lanthanoids, and actinoids, in the periodic table.

The carrier of a catalyst to be used in the present invention is not particularly limited, so long as it can stably support metal particles containing the above metal elements and it has excellent physical and chemical durability under the use conditions so that it can endure a long-term use. As such a carrier, for example, the following can preferably be used: (1) typical metal oxides such as silica, alumina, titania, zirconia, and magnesia; (2) mixed oxides such as silica-alumina, silica-titania, silica-zirconia, and titania-zirconia; (3) silica-basedcarrierscontaining various elements (e.g., one or more kinds of elements selected from aluminum, titanium, zirconium, lanthanoids, actinoids, or the like) supported on a silica carrier; (4) alumina-based carriers containing various elements (e.g., one or more kinds of elements selected from silicon, titanium, zirconium, lanthanoids, actinoids, or the like) supported on an alumina carrier; (5) zeolites and meso-porous silicate-based carriers with regular micro- and meso-pores, such as ZSM-5 and MCM-41; and (6) carbon materials such as activated carbons, fullerenes, and carbon nanotubes.

In these carriers, titania, zirconia, silica-titania, silica-zirconia, and silica-based carrier containing titanium and/or zirconia supported on a silica carrier can particularly preferably be used.

The catalyst of the present invention may contain other components, so long as the advantageous effects of the present invention are deteriorated. For example, it may contain alkali metals (Na, Ka, and the like), alkaline earths (Mg, Ca, Ba, and the like), and rare earths (La, Ce, and the like).

As the general properties, the catalyst may preferably have larger specific surface area, higher mechanical strength, and excellent chemical durability such as corrosion resistance. The specific surface area (as measured by the BET method) is usually not less than 10 m²/g, more preferably not less than 50 m²/g, and particularly preferably about 100 to 800 m²/g. When the specific surface area is less than 10 m²/g, metal particles are difficult to be supported, and even if supported, the amount of the metal particles supported is small or the particle diameter of the metal particles becomes large, by which the resulting catalyst is easy to become unsuitable for practical use.

In addition, the shape and size of the catalyst are not particularly limited, but they may appropriately be selected according to the reaction system. For example, when the catalyst is used in a fixed bed as the reaction system, there may preferably be used a catalyst having a spherical, cylindrical, or ring shape with a size of about 0.1 to 50 mm. When the catalyst is used in a fluidized bed or a suspended bed, there may preferably be used a catalyst having a spherical or crushed shape with a size of about 1 to 500 µm.

The method for the preparation of a catalyst is not particularly limited, so long as catalysts suitable for the method of the present invention can be prepared. The method for supporting Ag and/or Au (and, if necessary, any one or more kinds of group VIII elements) on a carrier is not particularly limited, but any of the previously well-known methods can be used. As the supporting method itself, any of the previously well-known methods can be utilized, such as coprecipitation method, deposition precipitation method, impregnating method, and chemical vapor deposition method. In these methods, preferred are the coprecipitation method, the deposition precipitation method, and the like, and particularly preferred is the deposition precipitation method.

A catalyst containing Ag and/or Au, and any one or more kinds of group VIII elements, supported on a carrier, as metal fine particle having an average particle diameter of not greater than 10 nm, is taken as an example, and a preferred method for the preparation of this catalyst will be described below. In the case where prepared is a catalyst containing only Ag or Au supported on a carrier, the preparation may be carried out according to the following preparation method.

The order that Ag and/or Au, and any one or more kinds of group VIII elements, are supported on a carrier is not particularly limited, and they may simultaneously be supported (simultaneous supporting method), or one is supported and the other is then supported (alternate supporting method). In particular, the method for simultaneously supporting both is preferred.

### (1) Simultaneous supporting method

A desired catalyst can be obtained by the addition of a carrier to an aqueous solution which contains a water-soluble compound containing Ag and/or Au and a water-soluble compound containing any one or more kinds of group VIII elements, dissolved therein, and by the deposition on the carrier of a precipitate containing Ag and/or Au and any one or more kinds of group VIII elements, and by the removal of the carrier having such a precipitate deposited thereon, and by the calcination of the carrier. If necessary, operations such as drying and reduction treatment may also be carried out.

The water-soluble compound containing Ag and/or Au is not particularly limited, so long as it is soluble in water. For example, as the compound containing Ag, there can be exemplified silver nitrate (AgNO₃), silver cyanide (AgCN), silver acetate (CH₃COOAg), silver lactate (CH₃CH (OH) COOAg), and the like. As the compound containing Au, there can be exemplified tetrachloroauric acid (HAuCl₄), sodium tetrachloroaurate (NaAuCl₄), potassium dicyanoaurate (KAu(CN)₂), diethylamine gold trichloride ((C₂H₅)₂NH-AuCl₃), gold cyanide (AuCN), and the like. These compounds may be used alone, or two or more kinds of them may also be used in combination.

The water-soluble compound containing group VIII elements is not particularly limited, so long as it is soluble in water. For example, there can be exemplified nitrates, sulfates, halides (chlorides, bromides, iodides), carboxylates (acetates, formates, and the like), acetylacetonates, ammine complexes, phosphine complexes, and the like. In case of Pd, there can be exemplified, for example, palladium oxide, palladium chloride, palladium bromide, palladium nitrate, palladium acetate, dichloro diamine palladium, dinitro diamine palladium, tetraammine palladium chloride, tetraammine palladium nitrate, tetraammine palladium hydrate, palladium acetylacetonate, dichloro tris(triphenylphosphine) palladium, and the like. In these compounds, nitrates, carboxylates, ammine complexes, and the like can more preferably be used, and ammine complexes can particularly preferably be used.

To produce the catalyst of the present invention, first of all, a compound containing Ag or Au and a compound containing a group VIII element are dissolved in water to prepare an aqueous solution. The method of dissolving these compounds is not particularly limited, but the respective compounds may be dissolved simultaneously, or after one is dissolved, the other may then be dissolved. The temperature at that time may be set at about 30°C to 80°C, for example.

The amount of the compound containing Ag or Au to be used, although it depends on the kind, specific surface area, and shape of a carrier, the amount of the carrier to be used, and the like, may preferably be such that the concentration of the gold compound in the aqueous solution is in the range of about 0.001 to 10 mmol/L. The above range of concentration makes it possible that the amount of the Ag and/or Au containing precipitate to be deposited becomes sufficient and the aggregation of the precipitate particles to be formed can be prevented, enabling the deposition of the precipitate in a state of fine particles. Therefore, there can extremely be reduced the amount of the compound remaining in the aqueous solution after the Ag and/or Au containing precipitate is supported on the carrier.

The amount of the group VIII element containing compound to be used, although it also depends on the kind, specific surface area, and shape of a carrier, the amount of the carrier to be used, and the like, may preferably be such that the concentration of the group VIII element containing compound in the aqueous solution is in the range of about 0.01 to 10 mmol/L. The above range of concentration makes it possible that the amount of the precipitate of the group VIII element containing compound becomes sufficient and the aggregation of the group VIII element containing compound particles can be prevented, enabling the deposition of the precipitate in a state of ultrafine particles. Therefore, there can extremely be reduced the amount of the compound remaining in the aqueous solution after the precipitate of the group VIII element containing compound is supported on the carrier.

The pH of the aqueous solution which contains the Ag and/or Au containing compound and the group VIII element containing compound is not particularly limited, but may preferably be set in the range of about 6 to 11. To adjust the pH of the aqueous solution in the above range, any compound showing alkalinity may appropriately be added to the aqueous solution. As such a compound, it is not particularly limited, but there can be used, for example, sodium carbonate, potassium carbonate, sodium hydroxide, potassium hydroxide, ammonia, and the like. These compounds may be added in solid state, or may be added after dissolved in water.

To an aqueous solution which contains Ag and/or Au containing compound and the group VIII element containing compound, surface active agents may be added for the purpose of improving the dispersibility of the components contained in the aqueous solution. As the surface active agent, there can be mentioned, but are not particularly limited to, for example, anionic surface active agents such as long chain alkyl sulfonic acids and salts thereof, long chain alkylbenzene sulfonic acids and salts thereof, long chain alkyl carboxylic acids and salts thereof, and aryl carboxylic acids and salts thereof; cationic surface active agents such as long chain alkyl quaternary ammonium salts; nonionic surface active agents such as polyalkylene glycols and polyoxyethylene nonylphenols. These surface active agents may be used alone, or two or more kinds of them may also be used in combination.

In the above exemplified surface active agents, anionic surface active agents and nonionic surface active agents are more preferred, and anionic surface active agents are particularly preferred. In the anionic surface active agents, more preferred are long chain alkyl sulfonic acid containing 8 or more carbon atoms and salts thereof, long chain alkylbenzene sulfonic acids containing 8 or more carbon atoms and salts thereof, long chain alkyl carboxylic acids containing 8 or more carbon atoms and salts thereof, aryl carboxylic acids and salts thereof, and the like. The amount of the surface active agents to be used may be set according to the kinds and combinations of the surface active agent, the gold compound, the group VIII element containing compound, and the carrier, and the like; therefore, it is not particularly limited, but may more preferably be such that the concentration of the surface active agent in the aqueous solution is in the range of 0.1 to 10 mmol/L.

Subsequently, the addition of a carrier to the aqueous solution, followed by stirring, may allow the carrier to be dispersed and suspended in the aqueous solution, and the precipitates of the Ag and/or Au containing compound and the group VIII element containing compound are deposited on the carrier. The temperature at that time may preferably be about 30°C to 80°C. The deposition time may usually be about 10 minutes to 5 hours.

Then, the carrier having the precipitate deposited on the surface thereof is calcined, if necessary, after washed with water, to obtain a desired catalyst. The temperature of calcination may be set at about 150°C to 800°C, preferably about 300°C to 800°C. The method of calcination is not particularly limited, but the calcination may be carried out in air or in an inert gas such as nitrogen gas, helium gas, or argon gas. The heating time may be set according to the temperature of heating; therefore, it is not particularly limited. The calcination may allow Ag and/or Au and any one or more kinds of group VIII elements to be firmly fixed on the surface of the carrier.

The above catalyst may be further subjected to the reduction treatment, if necessary. As the reduction treatment, the following two kinds of methods may preferably be utilized: (1) a method in which the catalyst is bought into contact with a gas containing a reducing gas such as hydrogen, carbon monoxide, and alcohol (e.g., methanol) at a temperature of about 100°C to 800°C, preferably about 150°C to 600°C; and (2) a method in which the reduction treatment is carried out using a reducing agent such as formalin, hydrazine, sodiumborohydride, and formic acid, in an aqueous medium at a temperature of about 0°C to 100°C, preferably about 30°C to 80°C, followed by drying at a temperature of about 50°C to 150°C.

### (2) Alternate supporting method

Either one of the Ag and/or Au containing compound and the group VIII element containing compound is deposited on a carrier, followed by drying and/or calcination, and subsequently, the other compound is deposited on the carrier, followed by calcination and, if necessary, reduction treatment, to obtain a desired catalyst.

As the method for supporting the Ag and/or Au containing compound on a carrier, the deposition precipitation method can be used under the same conditions as those of the simultaneous supporting method described above.

The method for supporting the group VIII element containing compound on a carrier is not particularly limited, but the supporting can be carried out according to any of the previously well-known methods. There can be mentioned, for example, the impregnating method, the ion exchange method, the chemical vapor deposition method, and the like. In these methods, the impregnating method can preferably be used. For example, after a carrier is added to a solution which contains the group VIII element containing compound dissolved therein, the collection of a solid content from the solution makes it possible that the group VIII element containing compound will be supported on the carrier.

The solution containing the group VIII element containing compound dissolved therein may be prepared by an appropriate combination of the compound and a solvent in which the compound can be dissolved. The solvent is not particularly limited, but water, organic solvent, and the like can be used. As the organic solvent, there can be mentioned, for example, alcohols, ketones, aromatic hydrocarbons, carboxylic acid esters, nitriles, and the like. It is particularly preferred to use at least one kind of water and alcohol (particularly, methanol and ethanol). Therefore, as the group VIII element containing compound, it is preferred to use any of the compounds which can be dissolved in water or in an alcohol.

The concentration of the group VIII element containing compound in the solution can appropriately be determined according to the kind of the compound, the kind of the solvent, and the like, but it may usually be set to be about 0.01 to 10 mmol/L.

The method for the collection of a solid content from the solution in which the group VIII element containing compound is dissolved is not particularly limited, but the collection may be carried out so that the group VIII element containing compound can be supported on the carrier. For example, it is preferred to remove the solvent by distillation using an evaporator or the like.

The Ag and/or Au containing compound and the group VIII element containing compound are sequentially supported on the carrier by the above method, followed by calcination, to obtain a catalyst in which the Ag and/or Au and the group VIII element are supported as metal particles. The conditions of calcination may be the same as those in the simultaneous supporting method described above. Further, it is preferred to carry out the subsequent reduction treatment, if necessary, in the same manner as in the simultaneous supporting method described above.

After either one of the Ag and/or Au containing compound and the group VIII element containing compound is deposited on the carrier, the carrier is dried or calcined before the other compound is deposited on the carrier. The conditions in this case are not particularly limited, but there can be used a method in which drying is carried out in air at room temperature or under heating at a temperature of about 150°C or lower; a method in which calcination is carried out under the same conditions as in the calcination method described above; and the like. Further, the reduction treatment as described above may be carried out, if necessary.

### 2. Starting material for oxidation

The present invention is characterized in that an alkyl substituent of an aromatic compound having the alkyl substituent is converted into an aldehyde group by oxidation with oxygen using the above catalyst. The use of this oxidation makes it possible to produce aromatic aldehyde compounds by oxidation of the alkyl substituent of the aromatic compound with oxygen in high yield and in high selectivity, which have not been attained so far.

The aromatic compound having an alkyl substituent, which is used as a starting material, is not particularly limited, so long as it is an aromatic compound having an alkyl group directly bonded to an aromatic ring. The term alkyl substituent as used in the present invention means a linear or branched alkyl group containing about 1 to 8 carbon atoms. In the present invention, alkyl groups such as methyl group, ethyl group, n-propyl group, and isopropyl group can be mentioned as the preferred groups. In these groups, a particularly preferred group is methyl group. In addition, the aromatic compound is not particularly limited, so long as it has an aromatic ring. There can be used aromatic compounds having an aromatic ring made of carbon and hydrogen, such as benzens, naphthalenes, and anthracenes; and an aromatic ring including oxygen, nitrogen, or sulfur, such as furans, pyridines, picolines, pyrroles, and thiophenes. These aromatic compounds may have various substituents other than alkyl groups.

Specific examples include (1) aromatic compounds having only an alkyl substituent(s), such as toluene, xylenes, ethylbenzene, isopropylbenzene, methyl cumenes, pseudocumene, and methylnaphthalenes; (2) aromatic compounds having a hydroxyl group in addition to an alkyl substituent, such as cresols and methylnaphthols; (3) aromatic compounds having an oxygen- or nitrogen-containing substituent(s) in addition to an alkyl substituent, such as anisole, methyl catechols, methyl benzoquinone, methylnaphthols, methylnaphthoquinones, nitrotoluenes, and methylanilines. In these compounds, (2) aromatic compounds having a hydroxyl group in addition to an alkyl substituent can provide high yield and high selectivity of the desired products; therefore, they can preferably be used. That is, preferably used are cresols such as o-, m-, and p-cresol; and methylnaphthols such as 2-methyl-1-naphthol, 4-methyl-1-naphthol, 7-methyl-1-naphthol, and 1-methyl-2-naphthol. In particular, cresols such as o-, m-, and p-cresol can preferably be used. These aromatic compounds having an alkyl substituent may be used alone, or two or more kinds of them may also be used in combination.

The aromatic compound as the starting material to be used may appropriately be selected depending on the kind of aromatic aldehyde compound as the desired product. For example, when aromatic aldehydes such as benzaldehyde, salicylaldehyde, and p-hydroxybenzaldehyde are produced, there may be used, as the starting material, aromatic compounds having a methyl substituent, such as toluene, o-cresol, and p-cresol, respectively. According to the production method of the present invention, aromatic ketone compounds can be synthesized. When aromatic ketones such as acetophenone, ethyl phenyl ketone, and diphenyl ketone are produced, there may be used, as the starting material, aromatic compounds having an alkyl substituent containing 2 or more carbon atoms, such as ethylbenzene, n-propylbenzene, and diphenylmethane, respectively.

### 3. Oxidation solvent

The oxidation reaction with oxygen of the present invention may preferably be carried out in an organic solvent as the field of reaction. The organic solvent which can be used is not particularly limited, so long as the reaction is carried out with good performance, but there are preferred solvents as shown below.

### 3-1. Case where the desired product obtained by the oxidation is aromatic aldehyde compound

When the desired product is an aromatic aldehyde compound, it is preferred to use an aprotic polar organic solvent. This is because the use of such a solvent can increase the yield and selectivity of the desired product. As the specific examples of the aprotic polar solvent, there can preferably be used: (1) ethers such as diethyl ether, diisopropyl ether, methyl t-butyl ether, and diethylene glycol diethyl ether; (2) cyclic ethers such as 1,4-dioxane, 1,3-dioxane, 1,3-dioxolane; (3) ketones such as acetone, methyl ethyl ketone, methyl isobutyl ketone, and cyclohexanone; (4) esters such as methyl acetate, ethyl acetate, butyl acetate, methyl propionate, ethyl isobutanate, methyl lactate, and dimethyl maleate; (5) halogen-containing hydrocarbons such as dichloromethane, chloroform, 1,2-dichloroethane; (6) S- and N-containing compounds such as dimethylsulfoxide, dimethylformamide, and dimethylacetamide; and the like. In these solvents, various ethers and ketones as recited in (1) to (3) can preferably be used, and the cyclic ethers as recited in (2) can particularly preferably be used. In addition, the amount of the organic solvent to be used may preferably be in the range of about 1 to 100, more preferably in the range of about 2 to 50, by molar ratio to the aromatic compound to be used as the starting material. Smaller amounts of solvent to be used may provide a reduction in selectivity, which case is not preferred. Greater amounts of solvent to be used may provide a reduction in productivity and a rise of the solvent recovery cost, which case is not preferred.

By the use of a non-aprotic solvent, that is, a solvent which has polarity but is protonic, such as acetic acid and methanol, the aromatic compound having an alkyl substituent as the starting material is oxidized into an aromatic carboxylic acid or an aromatic carboxylic acid ester, so that the selectivity of the desired aromatic aldehyde compound has a tendency to become decreased. As described later, when an aromatic carboxylic acid ester is the desired product, a primary alcohol is used as a solvent and a reactant by utilizing this tendency.

By the use of a non-polar solvent, that is, a solvent which is aprotic but is not polar, such as cyclohexane, benzene, the selectivity of the desired aromatic aldehyde compound has a tendency to become decreased as described above, and the activity (i.e., conversion) itself also has a tendency to become decreased.

In the oxidation in which case an aromatic aldehyde compound is the desired product, the above catalyst, an aromatic compound having an alkyl substituent, an organic solvent providing the field of reaction, and oxygen are essential components. Any other substances than the above-described components may exist at the same time during the reaction, so long as they do not adversely affect the reaction performance or catalyst life. The phrase "during the reaction" as use herein indicates any point of time from the start of reaction to the completion of reaction, and it is not particularly limited. In the substances which may exist at the same time in the reaction, there can be mentioned water and polyols as the substance providing favorable effects on the reaction performance. The presence of water and/or a polyol at the same time as the essential components in the reaction may provide an improvement in activity and selectivity in some cases. In particular, when an aromatic compound having an alkyl substituent and a hydroxyl group is used as the starting material, a polymer-like heavy component may be formed as a by-product of the oxidation in some cases. However, the presence of water and/or a polyol in the field of reaction can inhibit the formation of the above polymer-like heavy component, so that the effect of improving selectivity can be significantly exhibited.

In the oxidation, water is formed as a reaction product, and therefore, the presence of water at a constant amount may usually be observed in the reaction system. The amount of the water, which is present in the reaction, including water formed during the reaction, may preferably be in the range of about 0.1 to 100, more preferably in the range of about 1 to 50, by molar ratio to the aromatic compound as the starting material. Smaller amounts of water to be present may exhibit no significant effect, whereas greater amounts of water to be present may reduce yield, both of which cases are not preferred.

The term polyol specifically refers to an alcohol having two or more hydroxyl groups, but the kind thereof is not particularly limited. Usually, polyols having about 2 to 6 hydroxyl groups are preferred, and diols having two hydroxyl groups and triols having three hydroxyl groups are more preferred, and diols are particularly preferred. As the specific examples of the diols, there can be mentioned ethylene glycol, 1,2-propanediol, 1,3-propanediol, 1,2-butanediol, 1,3-butanediol, 2,3-butanediol, 1,2-cyclohexane diol, and the like. The amount of these diols to be present during the reaction may preferably be in the range of about 0.001 to 1, more preferably in the range of about 0.01 to 0.5, by molar ratio to the aromatic compound as the starting material. Smaller amounts of diol to be present may exhibit no significant effect, whereas greater amounts of diol to be present may reduce yield, both of which cases are not preferred.

### 3-2. Case where the desired product obtained by oxidation is aromatic carboxylic acid ester

When the desired product is an aromatic carboxylic acid ester, a primary alcohol serving as the reaction partner of the aromatic aldehyde compound may preferably be used for the organic solvent. As the primary alcohol, there can be mentioned aliphatic alcohols containing about 1 to 10 carbon atoms, such as methanol, ethanol, 1-propanol, and 1-octanol; diols containing about 2 to 10 carbon atoms, such as ethylene glycol and 1,4-butanediol; aliphatic unsaturated alcohols containing about 3 to 10 carbon atoms, such as allyl alcohol and methallyl alcohol; aromatic alcohols such as benzyl alcohol; and the like. Aliphatic alcohols containing 1 to 10 carbon atoms, and the like, may preferably be used. These alcohols may be used alone, or two or more kinds of them may also be used in combination.

According to the oxidation of the present invention, first of all, the α-position of an alkyl substituent is oxidized to form an aldehyde group, and this aldehyde group is then attacked by a primary alcohol to produce a corresponding carboxylic acid ester, finally providing an aromatic carboxylic acid ester having a carboxylic acid ester group directly bonded to the aromatic ring. That is, the use of alkylbenzenes, such as toluene, as the starting material, can provide benzoic esters; the use of alkylphenols, such as o-, m-, and p-cresol, as the starting material can provide hydroxybenzoic acids; and the use of alkylnaphthalenes, such as 1- and 2-methylnaphthalene, as the starting material, can provide naphthoic acid esters, respectively, as the corresponding aromatic carboxylic acid esters.

Therefore, an aromatic compound as the starting material and a primary alcohol to be reacted therewith may appropriately be selected depending on the desired aromatic carboxylic acid ester. For example, when methyl benzoate is a target product, toluene may be used as the aromatic compound and methanol may be used as the primary alcohol. When methyl salicylate is a target product, o-cresol and methanol may be used, respectively, as the aromatic compound and as the primary alcohol. When ethyl p-hydroxybenzoate is a target product, p-cresol and ethanol may be used, respectively, as the aromatic compound and as the primary alcohol. When n-propyl 6-hydroxy-2-naphthoate is a target product, 6-methyl-2-naphthol and 1-propanol may be used, respectively, as the aromatic compound and as the primary alcohol.

The ratio of aromatic compound and alcohol to be reacted is not particularly limited, but it may preferably be about 1 / 0.1 to 1 / 200, particularly preferably 1 / 1 to 1 / 50, by molar ratio of aromatic compound / alcohol. The above range makes it possible to produce an aromatic carboxylic acid ester with more efficiency.

When the desired product is an aromatic aldehyde compound, water and/or a polyol can be used as the substance providing favorable effects on the reaction performance. However, when the desired product is an aromatic carboxylic acid ester, a polyol, particularly ethylene glycol or 1,2-propylene glycol (most preferably, ethylene glycol), is extremely effective for improvement in activity and selectivity. The adsorption equilibrium of a polyol to the catalyst is fairly advantageous as compared with a primary alcohol; therefore, the alkyl substituent of an aromatic compound as the starting material is converted into an aldehyde group, and the polyol activated by the catalyst can then attack this aldehyde group more advantageously than the primary alcohol, resulting in the formation of a polyol ester. At that time, the ester interchange reaction will occur when the amount of the primary alcohol is in excess than that of polyol. As a result, an esterified product of the primary alcohol may finally be obtained as the main reaction product. Even in this case, the effect of inhibiting the formation of a polymer-like heavy component is also exhibited in the same manner as in the above-described case.

The amount of the polyol to be present during the reaction may preferably be in the range of about 0.001 to 1, more preferably in the range of about 0.01 to 0.5, by molar ratio to the aromatic compound as the starting material. Smaller amounts of polyol to be present may exhibit no significant effect, whereas greater amounts of polyol to be present may reduce yield, both of which cases are not preferred. In addition, when the polyol is a primary alcohol, but is not a primary alcohol corresponding to the desired product (i.e., it is not a primary alcohol required to be reacted), the selectivity of the desired product may be lowered in some cases; therefore, the use of such a polyol at a minimal amount is preferred.

In the substances other than the above polyol, which may be allowed to be present during the reaction, various kinds of solvents can preferably be used. Usually, in the practice of the present invention, it is preferred to use a primary alcohol at an excessive amount as compared with the aromatic compound as the starting material so that the primary alcohol is used both as the solvent and as the reaction partner. However, other solvents can also be added to the primary alcohol and used as a mixed solvent.

Specific examples of the solvent which can be used include, in addition to the above aprotic polar solvents, hydrocarbons such as hexane, cyclohexane, and octane; aromatic compounds such as benzene, toluene, and chlorobenzene; and the like. The use of such a compound as a solvent may provide an improvement in catalyst performance in some cases. The amount of such a solvent to be used may preferably be in the range of about 0.5 to 20, by molar ratio to the aromatic compound as the starting material. Smaller amounts of solvent to be added may exhibit no significant effect, which case is not preferred, whereas greater amounts of solvent to be added may cause the saturation of addition effect, leading to a waste of cost. The use of the above compound as a solvent may provide an improvement in catalyst performance in some cases.

### 4. Oxidation

In the present invention, the oxidation of an aromatic compound having an alkyl substituent with oxygen is carried out in the presence of an organic solvent and a catalyst. When the desired product is an aromatic carboxylic acid ester, a primary alcohol is also added to the reaction. The above reaction is usually carried out in a state consisting of three phases of vapor, liquid, and solid. That is, oxygen is introduced into a reactor as a gas, and an aromatic compound having an alkyl substituent, an organic solvent (mainly a primary alcohol when the desired product is an aromatic carboxylic acid ester), and a reaction product form a liquid phase, and a catalyst is used as a solid. Oxygen (oxygen gas) may be diluted with an inert gas such as nitrogen gas, argon gas, helium gas, and carbon dioxide gas. As an oxygen source, an oxygen-containing gas such as air may also be used. The system of the above reaction is not particularly limited, but any system can be applied, such as continuous system, batch system, or semibatch system. When a batch system is employed as the reaction system, the catalyst may be charged, together with the starting material, into a reactor. When a continuous system is employed as the reaction system, the catalyst may previously be filled into a reactor, or may continuously be charged, together with the starting material, into a reactor. The catalyst may have any of forms such as fixed bed, fluidized bed, and suspended bed.

The amount of the above catalyst to be used may appropriately be determined depending on the combination of a starting material, a solvent, and the like, the kind of catalyst, the reaction conditions, and the like. The reaction time is not particularly limited, but it may depend on the prescribed reaction conditions. Usually, it may be set to be about 0.5 to 20 hours as the reaction time or residence time (i.e., the amount of staying liquid in the reactor / the amount of liquid to be supplied). Various conditions such as reaction temperature and reaction pressure may appropriately be determined depending on the combination of a starting material and a solvent, and the kind of catalyst. The reaction temperature may usually be set to be about 0°C to 300°C, preferably about 20°C to 250°C, and more preferably about 50°C to 200°C. When the reaction temperature is set in such a range, the reactions can be allowed to progress with higher efficiency. The reaction pressure may be any of reduced pressure, ordinary pressure, or increased pressure. Usually, the reaction pressure may preferably be in the range of 0 to 5 MPa (gage pressure), particularly preferably 0 to 3 MPa. The amount of the catalyst to be used is not particularly limited, but it depends on the prescribed conditions. Usually, it may be set in the range of 0.001 to 2, preferably in the range of 0.005 to 1, and more preferably in the range of 0.01 to 5, by mass, relative to 1 as the amount of the aromatic compound having an alkyl substituent as the starting material.

After completion of the above reaction, the catalyst is separated from the reaction system, and the desired product formed (i.e., an aromatic aldehyde compound or an aromatic carboxylic acid ester) may be collected using any of the previously well-known means of separation and purification. In this case, the previously well-known methods of purification, such as distillation and crystallization, may usually be used. The separation of the catalyst may also be carried out according to any of the previously well-known methods. For example, when the reaction system is composed of the catalyst (solid content) and the reaction product (liquid component), the catalyst can be separated from the reaction product using any of the previously well-known methods of solid-liquid separation, such as filtration, centrifugation, and cyclone separation.

### Examples

The present invention will be more specifically described below; however, the present invention is not limited in any way by the following description.

### [Catalyst Preparation Example 1]

### Example of Au-Pd / Ti / SiO₂

First, to a commercially available silica carrier powder (Fuji Silysia Chemical Ltd.; "CARiACT Q-6") 100 g, was added 200 ml of a 2-propanol solution containing titanium isopropoxide (Wako Pure Chemical Industries, Ltd.) 17.8 g dissolved therein. After stirring well, the solvent was removed by distillation under heating to allow the titanium compound to be impregnated in and supported on the silica carrier. The silica carrier was then dried at 110°C for 10 hours, and calcined in air at 600°C for 4 hours.

Then, 500 ml of an aqueous chloroauric acid solution with a concentration of 18 mmol/L was adjusted to pH 10 using a 1N aqueous sodium hydroxide solution, while being kept at 65°C to 70°C. To this aqueous solution, was added 25 ml of an aqueous solution of tetraammine palladium hydroxide [(NH₃)₄Pd(OH)₂] (Pd content: 20 g/L; available from TOKURIKI HONTEN CO., LTD), into which 20 g of the above titanium-containing silica carrier was put, and the mixture was further stirred for 1 hour, while being kept at a temperature of 65°C to 70°C. The mixture was then allowed to stand, and the supernatant liquid was removed, after which 400 ml of ion exchanged water was added to the remaining solid, followed by stirring for 5 minutes at a room temperature and subsequent removal of the supernatant liquid. This washing operation was repeated 3 times. The solid obtained by filtration was dried at 110°C for 10 hours, and then calcined at 400°C in air for 3 hours to obtain a catalyst containing gold and palladium supported on the titanium-containing silica carrier (Au-Pd / Ti / SiO₂). The amounts of gold and palladium supported in the catalyst were 7.8% by mass and 2.5% by mass, respectively, as determined by fluorescent X-ray analysis. In addition, the observation of metal particle diameters by a transmission electron microscope showed that almost all of the metal species were highly dispersed on the carrier with a particle diameter of not greater than 10 nm and they apparently had an average particle diameter of not greater than 10 nm.

### [Catalyst Preparation Example 2]

### Example of Au-Pt / Ti / SiO₂

A catalyst containing gold and platinum supported on a titanium-containing silica carrier (Au-Pt / Ti /SiO₂) was obtained by the same operations as described in Catalyst Preparation Example 1, except that 36 ml of an aqueous solution of tetraammine platinum hydroxide [(NH₃)₄Pt(OH)₂] (Pt content: 10 g/L; available from Tanaka Kikinzoku Kogyo Co. Ltd.) was used instead of 25 ml of an aqueous solution of tetraammine palladium hydroxide in Catalyst Preparation Example 1. The amounts of gold and platinum supported in the catalyst were 8.0% by mass and 1.7% by mass, respectively, as measured by fluorescent X-ray analysis. In addition, the observation of metal particle diameters by a transmission electron microscope showed that almost all of the metal species were highly dispersed on the carrier with a particle diameter of not more than 10 nm and they apparently had an average particle diameter of not more than 10 nm.

### [Catalyst Preparation Example 3]

### Example of Au-Ir / Ti /SiO₂

A catalyst containing gold and iridium supported on a titanium-containing silica carrier (Au-Ir / Ti /SiO₂) was obtained by the same operations as described in Catalyst Preparation Example 1, except that 36 ml of an aqueous solution of tetraammine iridium nitrate [(NH₃)₆Ir(NO₃)₃] (Ir content: 10 g/L; available from Tanaka Kikinzoku Kogyo Co. Ltd.) was used instead of 25 ml of an aqueous solution of tetraammine palladium hydroxide in catalyst Preparation Example 1. The amounts of gold and iridium supported in the catalyst were 8.0% by mass and 1.5% by mass, respectively, as measured by fluorescent X-ray analysis. In addition, the observation of metal particle diameters by a transmission electron microscope showed that almost all of the metal species were highly dispersed on the carrier with a particle diameter of not more than 10 nm and they apparently had an average particle diameter of not more than 10 nm.

### [Production of Aromatic Aldehyde Compound]

### Experimental Example 1

### (Production of salicylic aldehyde using o-cresol as a starting material)

Into a 100-mL autoclave with a rotating stirrer were charged 1.8 g of o-cresol, 12 g of dioxane, 3 g of water, and 1.0 g of the catalyst containing gold and palladium supported (Au-Pd / Ti /SiO₂), which had been obtained above. The mixture was heated to 100°C under stirring, and nitrogen and oxygen were then charged at 0.3 MPa and 0.4 MPa by gage pressure, respectively, to start oxidation. While gradually adding oxygen, the total pressure was kept at 0.6 to 0.7 MPa, and the reaction was carried out at the same temperature for 3 hours. The autoclave was cooled and then opened. The catalyst was separated by filtration, and the contents were analyzed by gas chromatography. The conversion of o-cresol as the stating material was 67%, and the selectivity to salicylic aldehyde as the desired product was 65%.

### Experimental Example 2

### (Production of salicylic aldehyde using o-cresol as a starting material)

The reaction was carried out by the same operation as described in Experimental Example 1, except that the reaction time was changed from 3 hours to 2 hours. After the reaction, the analysis of the reaction product was carried out by the same operation. The conversion of o-cresol as the starting material was 46%, and the selectivity to salicylic aldehyde as the desired product was 75%.

### Experimental Example 3

### (Production of salicylic aldehyde using o-cresol as a starting material)

The reaction was carried out by the same operation as described in Experimental Example 1, except that the reaction time was changed from 3 hours to 5 hours. After the reaction, the analysis of the reaction product was carried out by the same operation. The conversion of o-cresol as the starting material was 88%, and the selectivity to salicylic aldehyde as the desired product was 52%.

### Experimental Example 4

### (Production of salicylic aldehyde using o-cresol as a starting material)

The reaction was carried out by the same operation as described in Experimental Example 1, except that 0.2 g of ethylene glycol was added and the reaction temperature was changed to 90°C. After the reaction, the analysis of the reaction product was carried out by the same operation. The conversion of o-cresol as the starting material was 72%, and the selectivity to salicylic aldehyde as the desired product was 70%.

### Experimental Example 5

### (Production of salicylic aldehyde using o-cresol as a starting material)

The reaction was carried out by the same operation as described in Experimental Example 4, except that dioxane was used at an amount of 24 g and water was used at an amonut of 6 g. After the reaction, the analysis of the reaction product was carried out by the same operation. The conversion of o-cresol as the starting material was 69%, and the selectivity to salicylic aldehyde as the desired product was 78%.

### Experimental Example 6

### (Production of p-hydroxybenzaldehyde using p-cresol as a starting material)

The reaction was carried out by the same operation as described in Experimental Example 4, except that o-cresol was changed to p-cresol. After the reaction, the analysis of the reaction product was carried out by the same operation. The conversion of p-cresol as the starting material was 71%, and the selectivity to p-hydroxybenzaldehyde as the desired product was 68%.

### Experimental Example 7

### (Production of benzaldehyde using toluene as a starting material)

The reaction was carried out by the same operation as described in Experimental Example 4, except that o-cresol was changed to toluene. After the reaction, the analysis of the reaction product was carried out by the same operation. The conversion of toluene as the starting material was 43%, and the selectivity to benzaldehyde as the desired product was 22%.

### Experimental Example 8

### (Production of salicylic aldehyde using o-cresol as a starting material)

The reaction was carried out by the same operation as described in Experimental Example 1, except that the solvent was changed from dioxane to 12 g of diethoxyethane. After the reaction, the analysis of the reaction product was carried out by the same operation. The conversion of o-cresol as the starting material was 33%, and the selectivity to salicylic aldehyde as the desired product was 89%.

### Experimental Example 9

### (Production of salicylic aldehyde using o-cresol as a starting material)

The reaction was carried out by the same operation as described in Experimental Example 1, except that the solvent was changed from dioxane to 12 g of acetone. After the reaction, the analysis of the reaction product was carried out by the same operation. The conversion of o-cresol as the starting material was 68%, and the selectivity to salicylic aldehyde as the desired product was 52%.

### Experimental Example 10

### (Production of salicylic aldehyde using o-cresol as a starting material)

The reaction was carried out by the same operation as described in Experimental Example 1, except that the solvent was changed from dioxane to 12 g of methanol. After the reaction, the analysis of the reaction product was carried out by the same operation. The conversion of o-cresol as the starting material was 67%, and the selectivity to salicylic aldehyde as the desired product was 39%.

### Experimental Example 11

### (Production of salicylic aldehyde using o-cresol as a starting material)

The reaction was carried out by the same operation as described in Experimental Example 1, except that the catalyst containing gold and platinum supported (Au-Pt / Ti / SiO₂), which had been obtained in Catalyst Preparation Example 2, was used as the catalyst instead of the catalyst containing gold and palladium supported (Au-Pd / Ti/ SiO₂). After the reaction, the analysis of the reaction product was carried out by the same operation. The conversion of o-cresol as the starting material was 22%, and the selectivity to salicylic aldehyde as the desired product was 58%.

### Experimental Example 12

### (Production of salicylic aldehyde using o-cresol as a starting material)

The reaction was carried out by the same operation as described in Experimental Example 1, except that the catalyst containing gold and iridium supported (Au-Ir /Ti / SiO₂), which had been obtained in Catalyst Preparation Example 3, was used as the catalyst instead of the catalyst containing gold and palladium supported (Au-Pd /Ti / SiO₂). After the reaction, the analysis of the reaction product was carried out by the same operation. The conversion of o-cresol as the starting material was 7%, and the selectivity to salicylic aldehyde as the desired product was 42%.

### Experimental Example 13

### (Production of salicylic aldehyde using o-cresol as a starting material)

The reaction was carried out by the same operation as described in Experimental Example 5, except that 0.2 g of 1, 3-propanediol was used instead of 0.2 g of ethylene glycol. After the analysis of the reaction product was carried out by the same operation. The conversion of o-cresol as the starting material was 71%, and the selectivity to salicylic aldehyde as the desired product was 74%.

### [Production of aromatic carboxylic acid ester]

### Experimental Example 14

### (Production of methyl salicylate using o-cresol as a starting material)

Into a 100-mL autoclave with a rotating stirrer were charged 1.8 g of o-cresol, 12 g of methanol, and 1.0 g of the catalyst containing gold and palladium supported (Au-Pd / Ti / SiO₂), which has been obtained above. The mixture was heated to 100°C under stirring, and nitrogen and oxygen were then charged at 0.3 MPa and 0.4 MPa by gauge pressure, respectively, to start oxidation. While gradually adding oxygen, the total pressure was kept at 0.6 to 0.7 MPa, and the reaction was carried out at the same temperature for 3 hours. The autoclave was cooled and then opened. The catalyst was separated by filtration, and the analysis of the reaction product was carried out by gas chromatography. The conversion of o-cresol as the stating material was 67%, the selectivity to salicylic aldehyde was 39%, and the selectivity to methyl salicylate as the desired product was 35%.

### Experimental Example 15

### (Production of methyl p-hydroxybenzoate using p-cresol as a starting material)

The reaction was carried out by the same operation as described in Experimental Example 14, except that o-cresol was changed to p-cresol. After the reaction, the analysis of the reaction product was carried out by the same operation. The conversion of p-cresol as the starting material was 67%, the selectivity to p-hydroxybenzaldehyde was 19%, and the selectivity to methyl p-hydroxybenzoate as the desired product was 65%.

### Experimental Example 16

### (Production of methyl benzoate using toluene as a starting material)

The reaction was carried out by the same operation as described in Experimental Example 14, that o-cresol was changed to toluene. After the reaction, the analysis of the reaction product was carried out by the same operation. The conversion of toluene as the starting material was 67%, the selectivity to p-hydroxybenzaldehyde was 5%, and the selectivity to methyl p-hydroxybenzoate as the desired product was 61%.

### Experimental Example 17

### (Production of methyl salicylate using o-cresol as a starting material)

The reaction was carried out by the same operation as described in Experimental Example 14, except that 0.5 g of ethylene glycol was charged and added to the starting material. After the reaction, the analysis of the reaction product was carried out by the same operation. After the reaction, the conversion of o-cresol as the starting material was 78%, the selectivity to salicylic aldehyde was 27%, and the selectivity to methyl salicylate as the desired product was 54%.

### Experimental Example 18

### (Production of methyl salicylate using o-cresol as a starting material)

The reaction was carried out by the same operation as described in Experimental Example 17, except that the reaction time was changed from 3 hours to 5 hours. After the reaction, the analysis of the reaction product was carried out by the same operation. The conversion of o-cresol as the starting material was 95%, the selectivity to salicylic aldehyde was 5%, and the selectivity to methyl salicylate as the desired product was 79%.

### Experimental Example 19

### (Production of methyl salicylate using o-cresol as a starting material)

The reaction was carried out by the same operation as described in Experimental Example 17, except that the catalyst containing gold and platinum supported (Au-Pt / Ti / SiO₂), which has been obtained in Catalyst Preparation Example 2, was used as the catalyst instead of the catalyst containing gold and palladium supported (Au-Pd / Ti / SiO₂). After the reaction, the analysis of the reaction product was carried out by the same operation. The conversion of o-cresol as the starting material was 31%, the selectivity to salicylic aldehyde was 4%, and the selectivity to methyl salicylate as the desired product was 51%.

### Experimental Example 20

### (Production of methyl salicylate using o-cresol as a starting material)

The reaction was carried out by the same operation as described in Experiment 17, except that the catalyst containing gold and iridium supported (Au-Ir /Ti /SiO₂), which had been obtained in Catalyst Preparation Example 3, was used as the catalyst instead of the catalyst containing gold and palladium supported (Au-Pd / Ti /SiO₂). After the analysis of the reaction product was carried out by the same operation. The conversion of o-cresol as the starting material was 9%, the selectivity to salicylic aldehyde was 5%, and the selectivity to methyl salicylate as the desired product was 58%.

### Experimental Example 21

### (Production of salicylic aldehyde using o-cresol as a starting material)

The reaction was carried out by the same operation as described in Experimental Example 18, except that 0.5 g of glycerol was used instead of 0.5 g of ethylene glycol. After the reaction, the analysis of the reaction product was carried out by the same operation. The conversion of o-cresol as the starting material was 92%, the selectivity to salicylic aldehyde was 5%, and the selectivity to methyl salicylate as the desired product was 76%.

### INDUSTRIAL APPLICABILITY

The oxidation method of the present inventionmakes it possible to obtain, in high yield and in high selectivity, aromatic aldehyde compounds, even when aromatic compounds having an alkyl substituent, which can easily be converted into aromatic carboxylic acids by oxidation, are used as starting materials, or to obtain aromatic carboxylic acid esters through these aromatic aldehyde compounds, by the use of a catalyst having moderate oxidating ability.

The aromatic aldehyde compounds obtained by the method of the present invention can be used for the same applications as those of aromatic aldehyde compounds obtained by the conventional technique. For example, hydroxy benzaldehydes are useful as intermediates for various drugs and as starting materials of resins.

The present invention, which makes it possible to produce aromatic carboxylic acid esters without allowing them to go through aromatic carboxylic acids, also has the advantageous feature that the reaction products can easily be obtained in higher purity particularly by its combination with purification though distillation. These aromatic carboxylic acid esters can be used for the same applications as those of aromatic carboxylic acid esters obtained by the conventional technique. For example, p-hydroxybenzoic acid esters are useful as starting materials of various liquid crystal polymers, as cosmetics, drugs and agricultural chemicals, and food additives, as the intermediates thereof, and the like. Salicylic acid esters are useful as flavoring agents and the like. The aromatic carboxylic acid esters according to the present invention, which can easily be obtained as products with low impurities particularly by purification through distillation, are extremely useful as starting materials of polymers and as drugs and agricultural chemicals, all of which are required to have high purity.

## Claims

1. A method for oxidation of an aromatic compound having an alkyl substituent, comprising reacting the aromatic compound having an alkyl substituent with an oxygen molecule to oxidize the alkyl substituent into an aldehyde group in a presence of a catalyst containing Ag and/or Au supported on a carrier.

2. The method for oxidation according to claim 1, wherein any one or more kinds of group VIII elements are further supported on the catalyst.

3. A method for producing an aromatic aldehyde compound, comprising reacting an aromatic compound having an alkyl substituent with an oxygen molecule to produce the aromatic aldehyde compound by the method for oxidation according to claim 1 or 2.

4. A method for producing an aromatic carboxylic ester, comprising reacting an aromatic compound having an alkyl substituent with an oxygen molecule to produce an aromatic aldehyde compound by the method for oxidation according to claim 1 or 2, and then reacting the aromatic aldehyde compound with a primary alcohol to produce the aromatic carboxylic acid ester.
